(19) Europäisches Patentamt
European Patent Office
Office européen des brevets

(11) **EP 3 697 245 B1**

(12) **FASCICULE DE BREVET EUROPEEN**

(45) Date de publication et mention
de la délivrance du brevet:
**09.03.2022 Bulletin 2022/10**

(21) Numéro de dépôt: **18803747.7**

(22) Date de dépôt: **16.10.2018**

(51) Classification Internationale des Brevets (IPC):
***A43B 3/00*** *(2022.01)*     ***A43B 7/00*** *(2006.01)*
***H04W 4/02*** *(2018.01)*     ***H04W 4/80*** *(2018.01)*

(52) Classification Coopérative des Brevets (CPC):
**A43B 7/005; A43B 3/34; H04W 4/023; H04W 4/80**

(86) Numéro de dépôt international:
**PCT/FR2018/052578**

(87) Numéro de publication internationale:
**WO 2019/077266 (25.04.2019 Gazette 2019/17)**

(54) **SYSTÈME ET PROCÉDÉ D'ANALYSE ET DE QUANTIFICATION DE LA POSTURE ET DE LA DÉMARCHE D'UN UTILISATEUR**

SYSTEM UND VERFAHREN ZUR GANGALNALYSE UND -QUANTIFIZIERUNG EINES BENUTZERS

SYSTEM AND METHOD TO ANALYZE AND QUANTIFY POSTURE AND GAIT OF A USER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.10.2017 FR 1771087**
         **26.04.2018 FR 1870497**

(43) Date de publication de la demande:
**26.08.2020 Bulletin 2020/35**

(73) Titulaire: **Zhor Tech**
**54000 Nancy (FR)**

(72) Inventeur: **OUMNIA, Karim**
**54000 Nancy (FR)**

(74) Mandataire: **A.P.I. Conseil**
**Immeuble Newton**
**4, rue Jules Ferry**
**64000 Pau (FR)**

(56) Documents cités:
**EP-A1- 2 458 338**     **WO-A1-98/00683**
**WO-A2-2014/141291**     **US-A- 4 703 445**
**US-A- 4 736 312**     **US-A1- 2010 070 316**
**US-A1- 2017 087 411**

**Description**

L'invention concerne le domaine de la chaussure ou plus généralement celui des articles chaussants. L'invention concerne plus particulièrement un boitier électronique pour semelles de chaussures connectées.

Plus précisément, il s'agit d'un système de mesure de données biomécaniques constitué de deux boitiers disposés chacun dans chacune des deux semelles de chaussure d'une même paire, destinés à recueillir et exploiter des informations sur la locomotion, le pattern de marche, la posture ou l'équilibre de l'utilisateur, ou très généralement la démarche, la course, ou l'activité de celui-ci.

**[Art antérieur]**

Les chaussures peuvent être à usage de détente, formel, sportif, médical, professionnel ou plus simplement récréatif. Une chaussure se compose principalement, d'une part, d'une semelle partie inférieure qui protège la plante des pieds, plus ou moins relevée à l'arrière par le talon et, d'autre part, de la tige, partie supérieure qui enveloppe le pied. Elle peut être limitée à la cheville ou être montante. La semelle peut se décliner en deux parties. Une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible. Dans ce cas particulier, cette semelle est aussi composée au moins d'une couche de semelle supérieure et d'une couche de semelle inférieure

Les semelles, qu'elles soient intérieures ou extérieures ou encore les chaussures au complet ont essentiellement pour rôle originel de protéger le pied du sol. Leur forme varie en fonction de la mode et de ses aléas pour laisser place à une multitude de produits dérivés et de fonctions. Néanmoins depuis plusieurs années maintenant, les nouvelles technologies de l'information accompagnent des besoins nouveaux et le monde de la chaussure fait partie de ce mouvement. Le développement de l'électronique a conduit à l'apparition de semelles et chaussures dites connectées, qui présentent des fonctions très diversifiées.

Parmi les semelles ou chaussures connectées, différents systèmes existent en vue de réaliser une pluralité de fonctions. Certains systèmes sont mis en œuvre grâce à une seule chaussure ou une seule semelle connectée. Par exemple, le document EP 1970671 et le document WO2011157870 décrivent chacun une chaussure intelligente conçue pour permettre à son utilisateur de commander différentes variables (distances parcourues, temps utilisé, calories consommées...) en vue de contrôler et améliorer ses performances sportives. Ce dispositif, basé sur un boitier unique, utilise un accéléromètre uniquement et pas de gyroscope, et ne permet pas de détecter le pattern de la marche, ni la posture. Ces systèmes ne permettent pas d'offrir une analyse fine de la démarche d'un utilisateur, ou de générer des données de qualités.

D'autres systèmes ont été réalisés, mettant en œuvre des capteurs positionnés sur deux chaussures ou deux semelles d'une même paire connectées. Dans ces systèmes, les capteurs sont souvent dispersés dans les chaussures ou les semelles ce qui d'une part altère le confort de l'utilisateur et d'autre part, entraine des surcoûts importants associés à la fabrication de l'article chaussant. Par exemple, le document US2017/0241797 décrit un appareil ayant pour but chez l'utilisateur de compter les pas et de distinguer la course de la marche normale par le biais d'un podomètre et d'un accéléromètre. Ce dispositif n'offre cependant pas d'informations sur des paramètres autres que la vitesse. De plus, l'appareil ne contient qu'un seul podomètre et un seul accéléromètre ce qui ne permet pas la génération de valeurs paramètre avancés de démarche. Dans un autre exemple, le document US2017/0188950, propose des chaussures équipées de capteurs de pression et d'un accéléromètre et qui permettent de délivrer à un smartphone connecté via Bluetooth des statistiques sur les activités physiques de leur porteur tels que le nombre de pas réalisés ou la façon dont se positionne le pied durant la marche, etc. Le document US2017/303827 quant à lui décrit un dispositif de semelles connectées comportant des capteurs pour l'étude de la démarche d'une personne. Doté d'un accéléromètre et d'un gyroscope, ce dispositif recueille des données biomécaniques, qui sont échangées entre les deux semelles et transmises à un terminal. Toutefois, les différents éléments de ce dispositif sont disséminés dans la semelle, notamment les capteurs de pression qui recueillent l'essentiel des données. Le document US 2016/324445 A1 divulgue un système d'analyse et de quantification de la posture et de la démarche d'un utilisateur tel que dans le préambule de la revendication 1.

Par ailleurs, ces dispositifs basés sur la présence de nombreux capteurs répartis dans la semelle (e.g. capteurs de pression) présentent une durée de vie plus faible et souvent une épaisseur relativement élevée pouvant limiter l'utilisation de ces semelles. En outre, les calculs ne sont généralement pas effectués en temps réel ce qui entraine des besoins élevés en termes de stockage de données et d'énergie.

Il existe des semelles utilisant des capteurs de pression censés identifier la posture, mais ces solutions techniques n'ont jamais permis à ce jour d'avoir un produit commercialisable et efficace.

Il existe donc un besoin pour un nouveau système équipé de deux chaussures ou semelles connectées permettant de rassembler des capteurs suffisant pour générer des données brutes de qualités afin d'analyser finement la démarche tout en étant compact, autonome et résistant.

**[Problème technique]**

L'invention a pour but de remédier aux inconvénients de l'art antérieur. En particulier, l'invention a pour but de permettre à l'utilisateur d'accéder, particulièrement au travers du boitier électronique installé dans chacune de ses semelles connectées, à des informations sur la posture exacte de ses pieds et plus généralement sur sa démarche ou son activité.

En outre, l'invention a pour but de proposer une solution pour la quantification de la démarche qui soit compacte, résistante, d'autonomie élevée et qui soit de préférence configurée pour réaliser un traitement des données qui ne consomme qu'un minimum n'énergie.

**[Brève description de l'invention]**

A cet effet, l'invention concerne donc un système d'analyse et de quantification de la posture et de la démarche d'un utilisateur, caractérisé en ce qu'il comporte deux boitiers électroniques aptes à être intégrés dans une paire de semelles, un premier boitier étant apte à être intégré dans une première semelle et un second boitier étant apte à être intégré dans une seconde semelle, chaque boitier comprenant :

- une plateforme inertielle configurée pour générer un ensemble de données sur la posture, l'activité ou la démarche d'un utilisateur de la paire de semelles ;
- un module de traitement de données configuré pour prétraiter l'ensemble de données recueillies en fonction d'algorithmes prédéfinis et générer une information sur la posture, l'activité ou la démarche d'un utilisateur de la paire de semelles ;
- un module de stockage de données configuré pour mémoriser l'information générée par le module de traitement ;
- un moyen de communication configuré de façon à ce qu'un boitier électronique d'au moins une des semelles soit apte à transmettre l'information générée sur l'utilisateur à un terminal externe et/ou à l'autre boitier de l'autre seconde semelle ; et
- une source d'énergie, ledit système étant caractérisé en ce que les boîtiers sont configurés de telle sorte que les données brutes générées par la plateforme inertielle d'un premier boîtier subissent une première étape de traitement par le module de traitement de données du premier boîtier puis que les données traitées soient transférées au second boîtier où elles seront à nouveau traitées par le module de traitement de données du second boîtier.

Un tel système permet de suivre la démarche d'un utilisateur de façon fiable. En effet, la présence d'une paire de semelle comportant chacune un boitier protégeant une plateforme inertielle donne la possibilité de suivre de façon indépendante le mouvement de chacun des pieds. La plateforme inertielle va analyser, en au moins trois dimensions, la posture, les mouvements, la locomotion, l'équilibre et l'environnement de l'utilisateur, et plus généralement tout ce qui est lié à l'activité de ses pieds ou qui sera qualifié comme étant sa démarche. La plateforme inertielle va pouvoir non seulement constater les différentes positions mais également relever des carences ou des anomalies qui apparaissent au niveau de la locomotion, du pattern, ou plus généralement de la marche de l'utilisateur. En outre, le présent boitier électronique rassemblant l'ensemble des composants électronique nécessaires à un fonctionnement autonome, tels que tous les capteurs, y compris des modules de calcul et une source d'énergie, cela permet d'augmenter la robustesse du système. Ce boitier peut avantageusement être unique, compact et miniaturisé.

En outre, contrairement aux systèmes proposés dans l'art antérieur, le calcul est ici réalisé au niveau de la semelle par l'intermédiaire d'un module de traitement de données pouvant correspondre au micrologiciel (« firmware » en terminologie anglo-saxonne) d'une carte électronique. De cette façon, les données sont transformées quasiment en temps réel au niveau du boitier électronique, comparées puis peuvent ensuite être transférées pour leur visualisation sur un terminal externe. Un tel système permet de réduire la sollicitation de la mémoire du module de stockage et peut alors permettre d'augmenter l'autonomie du système.

Les boitiers sont configurés de sorte que des données brutes générées par la plateforme inertielle d'un premier boitier subissent une première étape de traitement par le module de traitement de données du premier boitier puis que les données traitées soient transférées au second boitier où elles seront à nouveau traitées par le module de traitement de données du second boitier. Ainsi, en particulier, les boitiers sont paramétrés de sorte qu'un premier boitier reçoit les données de sa semelle et les transmet à un second boitier, qui traite les données reçues en les comparant à ses propres données et génère une information sur la démarche de l'utilisateur ou la posture de ses pieds, information qui est ensuite transmise par l'un des boitiers au terminal externe en temps réel ou de manière différée.

Selon d'autres caractéristiques optionnelles de l'invention:

- les modules de traitements de données contenus dans le premier et le second boitier, sont configurés pour comparer des données brutes générées par la plateforme inertielle du boitier respectif à des motifs prédéterminés et pour générer des valeurs de similarités des données brutes avec les motifs prédéterminés, lesdits motifs prédéterminés correspondants à une catégorie de mouvement prédéterminée, l'un des modules de traitements étant en outre configuré pour sélectionner une catégorie de mouvement représentative des données brutes générées sur une période de temps, à partir des valeurs de similarités du premier boitier et celles du second boitier. Ainsi, il est possible d'identifier une catégorie de mouvement avec un minium de consommation d'énergie, et de mémoire.

- chacun des boitiers contient une carte électronique dotée d'une plateforme inertielle, constituée d'au moins un accéléromètre et d'au moins un gyroscope, et qui peut être complétée par d'autres capteurs, notamment un magnétomètre, un baromètre et un altimètre.
- chacun des boitiers, outre la carte électronique et une source d'énergie, peut également être connecté à un GPS et/ou à tous types de capteurs, notamment des capteurs physiologiques, capteurs de pression, de température ou tout système de climatisation placés dans la semelle.
- chacun des boitiers est conçu de manière à pouvoir communiquer avec le second boitier et/ou directement avec le terminal externe par exemple par des signaux d'ondes courtes ou hautes fréquences de type Bluetooth ou ANT+. Cela par exemple afin d'échanger ses propres informations sur la posture, le mouvement et l'activité de son pied, dont il a reçu les données via sa plateforme inertielle et les capteurs de sa semelle. En particulier, le premier boitier électronique de la première semelle est configuré pour transmettre des données provisoires sur l'utilisateur au second boitier de l'autre semelle et ledit second boitier est configuré pour transmettre de l'information sur la démarche à un terminal externe (20).
- chaque boitier électronique comporte au moins un plot de soutien, de préférence au moins deux plots de soutien.
- chaque boitier électronique pèse moins de 20 grammes.
- chaque boitier électronique présente une épaisseur inférieure ou égale à 7 mm.
- chaque boitier électronique présente une surface sur sa face la plus grande inférieure ou égale à 10 cm$^2$.
- chacun des boitiers électroniques comporte une enveloppe extérieure, ladite enveloppe extérieure étant essentiellement constituée d'un matériau thermoplastique, par exemple un matériau composite thermoplastique, lui permettant de résister à de fortes contraintes mécaniques, (évaluées et testées en condition d'utilisation - boitier introduit dans une semelle ou une chaussure) correspondant au minimum à 100 000 impacts de 1000 N à une fréquence de 1Hz ou 100 000 impacts de 3000 N à une fréquence de 2,6 Hz, lesdits boitiers étant par ailleurs résistants à la poussière et l'humidité à un niveau au minimum IP56.
- le système selon l'invention permet d'associer une ou plusieurs applications Android, IOS ou autres à un partage sécurisé des données avec l'utilisateur via le terminal externe.
- la source d'énergie du boitier électronique peut être une batterie rechargeable, dont la recharge peut être réalisée selon différentes technologies :

- par chargeur, avec un connecteur affleurant au niveau de la semelle;
- avec un dispositif de recharge mécanique intégré à la semelle, comme par exemple un dispositif piézoélectrique apte à fournir une énergie électrique à partir de la marche ;
- avec un dispositif sans contact, par exemple par induction ; ou
- avec un dispositif photovoltaïque.

Selon un autre aspect, l'invention porte sur un procédé de quantification de la posture et de la démarche d'un utilisateur susceptible d'être mis en œuvre au sein d'un système selon l'invention. Par exemple mis en œuvre au sein d'un système comportant deux boitiers électroniques intégrés dans une paire de semelles, un premier boitier étant intégré dans une première semelle et un second boitier étant intégré dans une seconde semelle d'une même paire de semelle, chaque boitier comprenant : une plateforme inertielle, un module de traitement de données, un module de stockage de données configuré pour mémoriser l'information générée par le module de traitement, un moyen de communication et une source d'énergie, comportant un premier boitier intégré dans une première semelle et un second boitier intégré dans une seconde semelle d'une même paire de semelle, ledit procédé comprenant :

- Une étape de génération de données brutes, par des plateformes inertielles contenues dans le premier et le second boitier, lesdites données brutes étant générées pour une période de temps et étant fonction de la démarche d'un utilisateur,
- Une étape de prétraitement des données brutes, par les modules de traitement de données contenus dans le premier et le second boitier, comprenant la comparaison des données brutes générées à des motifs prédéterminés et la génération de valeurs de similarités des données brutes avec les motifs prédéterminés, lesdits motifs prédéterminés correspondants à une catégorie de mouvement prédéterminée,
- Une étape de communication entre le premier et le second boitier, comprenant la transmission, par un module de communication, des valeurs de similarités du second boitier au premier boitier, et
- Une étape de sélection, par le premier boitier, d'une catégorie de mouvement représentative des données brutes générées sur la période de temps, à partir des valeurs de similarités du premier boitier et celles du second boitier.

Selon d'autres caractéristiques optionnelles du procédé :

- il comprend en outre une étape d'incrémentation d'un compteur associé à la catégorie de mouvement sélectionnée et une mémorisation de la nouvelle valeur dudit compteur,

- l'étape de prétraitement comporte une sous étape de calcul de valeurs provisoires de paramètres de démarche par le premier et le second boitier. En particulier, l'étape de prétraitement comporte une sous étape de calcul de valeurs provisoires de paramètres de démarche par le premier et le second boitier, lesdites valeurs provisoires de paramètres de démarche étant transmise, par le module de communication, du second boitier au premier boitier et le procédé comporte alors une étape de comparaison des valeurs provisoires de paramètres de démarche du second boitier à celle du premier boitier de façon à générer une valeur consolidée de paramètres de démarche. En outre, dans ce cas, le procédé peut également comporter une étape de modification de compteurs associés aux paramètres de démarche et une mémorisation de la nouvelle valeur desdits compteurs.

- il comprend une étape de traitement des valeurs provisoires de paramètres de démarche du second boitier et du premier boitier de façon à sélectionner des valeurs provisoires de paramètres de démarche à conserver.

- il comprend une étape préalable d'apprentissage comprenant une définition d'une pluralité de motifs prédéterminés de démarche d'un utilisateur. Ces motifs prédéterminés de démarche sont avantageusement associés à des catégories de mouvement tels que par exemple la marche, la montée de marche, un saut...

D'autres avantages et caractéristiques de l'invention apparaitront à la lecture de la description suivante donnée à titre d'exemple illustratif et non limitatif, en référence aux Figures annexées qui représentent :

- Figure 1, une vue en coupe longitudinale du dessus de deux semelles chacune contenant une cavité qui va laisser place à un boitier, chacune des antennes des boitiers étant située sur l'arrête extérieur vis à vis de chaque pied, selon un mode de réalisation de l'invention.

- Figure 2, un boitier électronique ouvert vu du dessus comprenant notamment une carte électronique, une batterie rechargeable, un connecteur et une antenne.

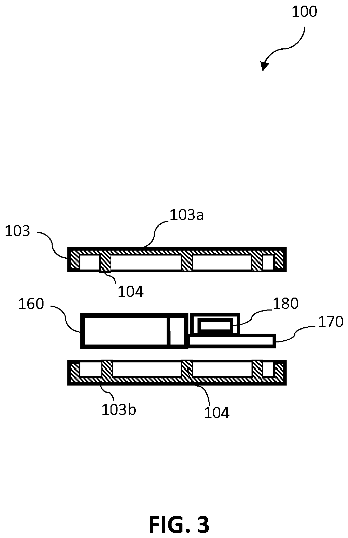

- Figure 3, un boitier électronique, en vue éclatée en coupe de profil, comprenant notamment une batterie rechargeable, une carte électronique ainsi qu'une enveloppe extérieure en deux parties.

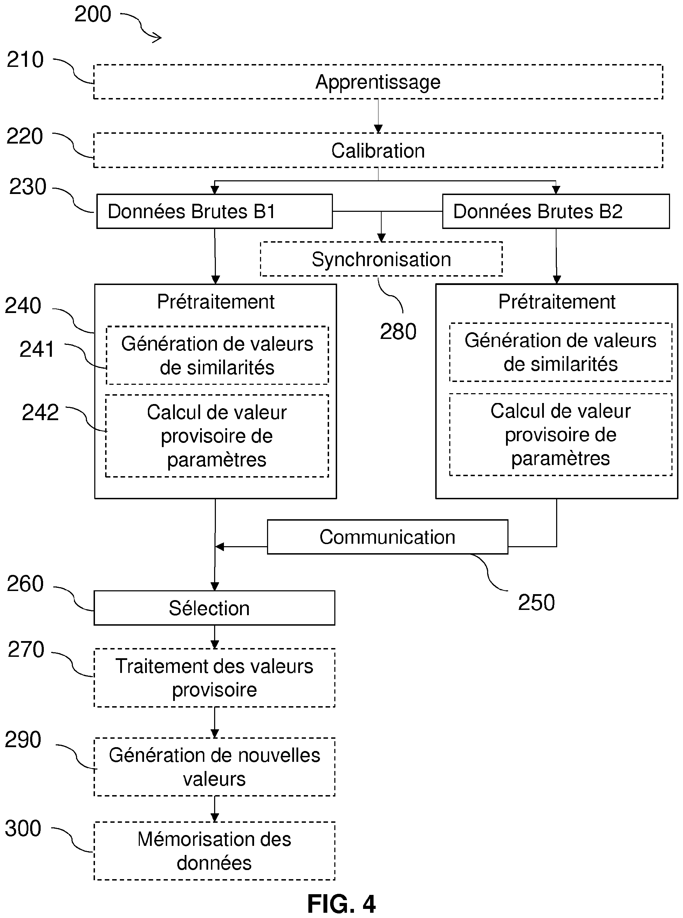

- Figure 4, un schéma représentatif du procédé selon un mode de réalisation de l'invention

Des aspects de la présente invention sont décrits en référence à des organigrammes et / ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. A cet égard, chaque bloc dans les organigrammes ou blocs-diagrammes peut représenter un système, un dispositif, un module ou un code, qui comprend une ou plusieurs instructions exécutables pour mettre en œuvre la ou les fonctions logiques spécifiées. Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et / ou de l'organigramme, et des combinaisons de blocs dans les schémas de principe et / ou l'organigramme, peuvent être mis en œuvre par des systèmes matériels spéciaux qui exécutent les fonctions ou actes spécifiés ou effectuer des combinaisons de matériel spécial et d'instructions informatiques.

## [Description de l'invention]

On entend par « semelle » un objet permettant de séparer le pied de l'utilisateur du sol. Une chaussure peut comporter une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible.

Dans la suite de la description, la « démarche » au sens de l'invention correspond à la posture, les mouvements, la locomotion, et l'équilibre de l'utilisateur. L'équilibre correspond notamment à l'équilibre postural lié à la stabilité du corps et plus particulièrement à la stabilité du centre de gravité d'un utilisateur. Néanmoins il peut intégrer aussi bien l'équilibre statique au l'équilibre dynamique.

La « quantification de la démarche » correspond, au sens de l'invention, à l'attribution d'une ou de plusieurs valeurs par exemple un score, un classement ou une note à une trajectoire ou un déplacement d'un pied d'un utilisateur. Cette quantification de la démarche permet d'obtenir une ou plusieurs valeurs de paramètre biomécanique représentatives de la démarche et peut être réalisée sur la base de nombreuses échelles de tailles différentes (e.g. 1, 5, 10, 100) linéaires ou non.

Par « paramètre biomécanique » et plus particulièrement par « paramètre calculé à partir de données de mouvement », on entend au sens de l'invention le résultat d'une transformation de la trajectoire mesurée d'un pied d'un utilisateur en une ou plusieurs valeurs.

Par « modèle » ou « règle » ou « algorithme » il faut comprendre au sens de l'invention une suite finie d'opérations ou d'instructions permettant de calculer une valeur par l'intermédiaire d'un classement ou d'un partitionnement des données au sein de groupes préalablement définis Y et d'attribuer un score ou de hiérarchiser une ou plusieurs données au sein d'un classement. La mise en œuvre de cette suite finie d'opérations permet par exemple d'attribuer une étiquette Y à une observation décrite par un ensemble de caractéristiques ou paramètres X grâce par exemple à la mise en œuvre d'une fonction f susceptible, de reproduire Y ayant observé X.

$$Y = f(X) + e$$

où e symbolise le bruit ou erreur de mesure

Par « pattern », on entend la manière dont l'utilisateur déroule son pied à l'occasion de sa marche ou de sa course.

On entend par « traiter », « calculer », « déterminer », « afficher », « transformer », « extraire » « comparer » ou plus largement « opération exécutable », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou tout autre séquence d'instructions conçues pour l'exécution sur un système informatique.

On entend par « composite plastique », au sens de l'invention, un matériau multi-composants comprenant au moins deux composants non miscibles dans lequel au moins un composant est un polymère (thermoplastique ou thermodurcissable) et l'autre composant peut être un renfort tel qu'un renfort fibreux.

On entend par « renfort fibreux » ou « substrat fibreux », au sens de l'invention, plusieurs fibres, des stratifils unidirectionnels ou un mat à filament continu, des tissus, des feutres ou des non-tissés qui peuvent être sous la forme de bandes, nappes, tresses, mèches ou pièces.

Par « polymère », on entend soit un copolymère soit un homopolymère. On entend par « copolymère », un polymère regroupant plusieurs unités monomères différentes et par « homopolymère », un polymère regroupant des unités monomères identiques.

On entend par « polymère thermoplastique », au sens de l'invention, un polymère généralement solide à température ambiante, pouvant être cristallin, semi-cristallin ou amorphe, et qui se ramollit lors d'une augmentation de température, en particulier après passage de sa température de transition vitreuse (Tg) et s'écoule à plus haute température. Des exemples de thermoplastiques sont, par exemple : le polyéthylène basse densité (PEHD), le polyéthylène téréphtalate (PET) ou le polychlorure de vinyle (PVC).

On entend par « Polymère thermodurcissable » une matière plastique qui se transforme de manière irréversible par polymérisation en un réseau polymère insoluble.

On entend par « amovible » la capacité à être détachée, enlevée ou démontée aisément sans avoir à détruire des moyens de fixation soit parce qu'il n'y a pas de moyen de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

Dans cette description et même avant, les mêmes références sont utilisées pour désigner les mêmes éléments.

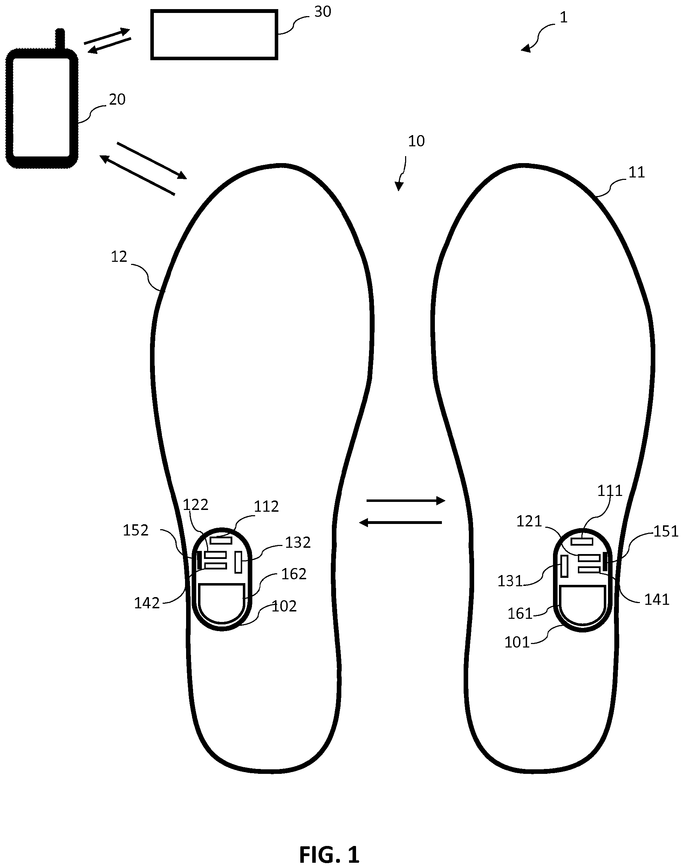

Les dispositifs ou systèmes existants présentent généralement une pluralité de capteurs (e.g. capteurs de pression) répartis dans la chaussure et/ou semelle. Une telle répartition des capteurs entraine une réduction de la robustesse du système. En outre, ces dispositifs ou systèmes sont généralement destinés à produire des données brutes qui sont ensuite analysées au niveau d'un terminal externe. Face à ces insuffisances, l'inventeur a développé un système 1 pour la quantification de la démarche d'un utilisateur tel que schématisé sur la Figure 1.

Pour rappel, les deux pieds contiennent le quart de tous les os du corps humain. Dans chaque pied, il est possible d'identifier 26 os, 33 muscles, 16 articulations et 107 ligaments. Les pieds portent le poids du corps en position debout et permettent la locomotion, assurant un rôle primordial dans l'équilibre, l'amortissement et la propulsion. Les pieds effectuent aussi plusieurs types de mouvements. En outre, les pieds comptent près de 7 200 terminaisons nerveuses, de sorte que toutes les maladies et autres troubles notamment neurologiques sont perceptibles directement ou indirectement au niveau de nos pieds et, d'autre part, peuvent être détectés à partir de notre manière de marcher ou de nous mouvoir.

Pour poursuivre cet objectif, l'invention consiste en une technologie autonome insérée dans un boitier compact et miniaturisé de quelques grammes, qui est inséré dans chacune des deux semelles intérieures et/ou extérieure de chaussures d'une même paire.

Ce sont les choix décrit si après qui ont permis aux inventeurs d'obtenir un boitier compact et miniaturisé de quelques grammes qui est néanmoins capable de résister à des pressions et forces répétées dans le cadre de marche, courses sauts ou autres activités sportives.

**Selon un premier aspect**, l'invention porte sur un dispositif intégré dans un boîtier électronique inséré dans chacune des deux semelles intérieures et/ou extérieures de chaussures d'une même paire et récoltant particulièrement des données sur la locomotion, le pattern ou l'équilibre de l'utilisateur, ou très généralement la marche ou l'activité de celui-ci, qu'il communique au boîtier de l'autre semelle et à un terminal externe, notamment par des signaux ondes courtes ou hautes fréquences de type Bluetooth, dans le but d'analyser et déterminer la posture/le mouvement/l'activité de chacun des pieds, ledit boîtier comprenant notamment une carte électronique de type PCBA et une source d'énergie, caractérisé en ce que ce boîtier, qui dispose d'une mémoire interne de stockage des données, traite et échange ses données en fonction d'algorithmes prédéfinis, avant de les transmettre au terminal externe pour traitement par une application dédiée.

L'invention porte également sur un système de recueil et d'exploitation des informations sur la locomotion, le pattern ou l'équilibre de l'utilisateur, ou très généralement la marche ou l'activité de celui-ci, caractérisé en ce qu'il comprend un boîtier électronique inséré dans chacune des deux semelles intérieures et/ou extérieures de chaussures d'une même paire et récoltant des données qu'il communique au boîtier de l'autre semelle et à un terminal externe, notamment par des signaux ondes courtes ou hautes fréquences de type Bluetooth, dans le but d'analyser et déterminer la posture/le mouvement/l'activité de chacun des pieds, ledit boîtier qui, comprenant notamment une carte électronique de type PCBA et une source d'énergie et disposant d'une mémoire interne de stockage des données, traite et échange ses données en fonction d'algorithmes prédéfinis, avant de les transmettre au terminal externe pour traitement par une application dédiée.

En particulier, l'invention porte sur un système comportant deux boitiers tels que décrits ci-dessous. En particulier, elle porte sur un système comportant deux boitiers électroniques 100, 101, 102, aptes à être intégrés dans une paire 10 de semelles, un premier boitier étant apte à être intégré dans une première semelle et un second boitier étant apte à être intégré dans une seconde semelle.

En particulier, le système 1 selon l'invention peut comporter une paire 10 de semelles comportant les boitiers électroniques 100, 101, 102 selon l'invention et éventuellement un terminal externe 20.

Les semelles 11, 12 utilisables dans le cadre du système 1 selon l'invention peuvent par exemple correspondre à des semelles extérieures ou à des semelles intérieures, de chaussures. Ces semelles peuvent être amovibles ou être intégrées de manière permanente au semelage des chaussures.

Classiquement, les semelles 11, 12 composants ladite paire 10 de semelles, comportent chacune un boitier électronique 100,101,102. Comme présenté sur la figure 1, le boitier électronique 101,102 est de préférence positionné au niveau d'une portion médiane de la semelle.

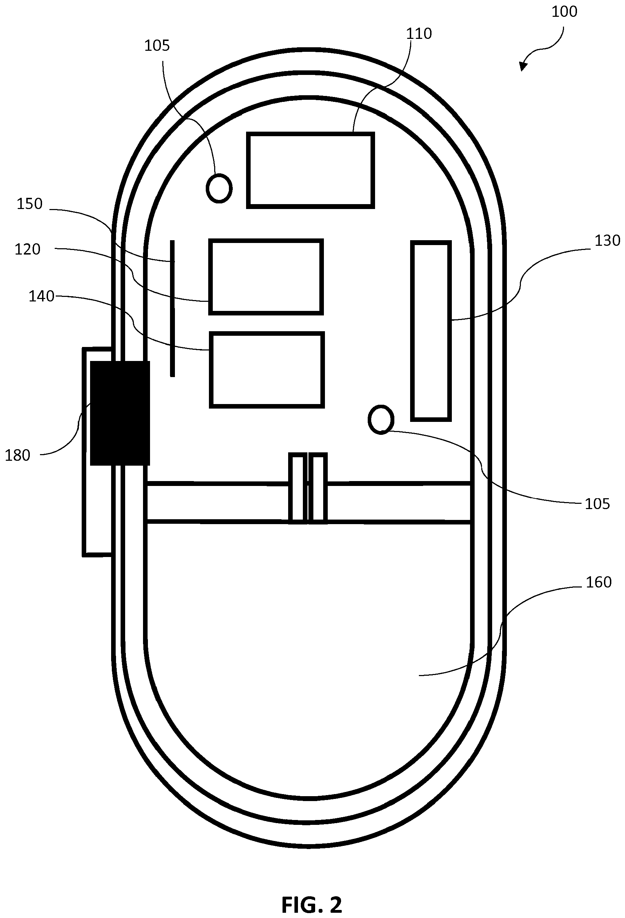

Un boitier électronique 100 selon l'invention est détaillé dans la figure 2. Ne pesant que quelques grammes et étant de dimension réduite, ce boitier électronique 100 se loge de façon peu encombrante dans n'importe quelle semelle intérieure et/ou extérieure. Ce faible volume limite l'impact sur le confort de l'utilisateur et présente l'avantage d'optimiser les coûts de production en rendant moins onéreux et plus simple l'intégration de cette technologie dans la semelle lors du processus industriel.

Le choix de la matière de ce boitier électronique est réalisé de façon à assurer sa solidité ainsi que la possibilité de l'insérer dans une semelle. En effet, il convient de pouvoir fabriquer un produit qui puisse d'une part, résister au poids d'une personne et, d'autre part, être facilement inséré dans une semelle ou une chaussure. Allier miniaturisation et résistance du boitier est un véritable challenge : il a fallu réaliser de nombreux prototypes avant de déterminer la matière qui permette d'insérer un tel boitier dans une semelle, sans altérer le confort de celle-ci.

Avantageusement, chaque boitier électronique 100 comporte une enveloppe extérieure 103, ladite enveloppe extérieur étant essentiellement constituée d'un matériau de type composite plastique sélectionné parmi : un matériau composite thermoplastique ou un matériau composite thermodurcissable. De façon préférée, le matériau utilisé est à base de thermoplastique tel que le polycarbonate et peut comprendre du nylon ou de la fibre de verre. En effet, le polycarbonate a l'avantage de pouvoir être thermoformé, résistant mécaniquement et peu inflammable ce qui est avantageux lors de la soudure par ultrason détaillée ci-après.

Le choix d'un matériau en polymère, par exemple en composite plastique, permet d'allier à la fois légèreté, efficacité de la transmission du signal et surtout solidité. Avantageusement, l'enveloppe est arrondie, autrement dit, elle ne possède pas d'angle inférieur à 95°.

Cette forme d'enveloppe permet d'améliorer le confort de l'utilisateur.

Ainsi, chaque boitier électronique est de préférence léger et pèse par exemple moins de 20 grammes, moins de 10 grammes, de préférence moins de 8 grammes et de façon plus préférée moins de 6 grammes. En outre, il peut présenter une épaisseur inférieure à 7 mm, ou inférieure à 5 mm, de préférence inférieure à 4 mm et de façon plus préférée inférieure à 3 mm. Cela lui permet de s'intégrer facilement dans une chaussure/semelle sans altérer le confort de l'utilisateur dans sa chaussure. Enfin, chaque boitier électronique présente par exemple moins de 10 cm$^2$ de surface sur sa face la plus grande, de préférence moins de 5 cm$^2$ de surface sur sa face la plus grande, de plus préférée moins de 4 cm$^2$ et de façon encore plus préférée moins de 3 cm$^2$.

De façon préférée, l'enveloppe extérieure 103 du boitier électronique 100 comporte une partie supérieure 103a et une partie inférieure 103b qui sont soudées. Une telle soudure, par exemple une soudure par ultrasons, permet d'augmenter la résistance à l'eau du boitier électronique. Alternativement, la partie supérieure 103a et la partie inférieure 103b peuvent être séparées par un joint en polymère et maintenues par des moyens de fixation amovibles. Ainsi, chaque boitier électronique peut comporter une enveloppe extérieure formée en deux parties et un joint venant se positionner entre deux parties de l'enveloppe extérieure.

De préférence de forme arrondie pour augmenter sa résistance mécanique, il doit être assemblé de telle sorte à maintenir une étanchéité parfaite et rendre l'intérieur contenant la carte électronique et la source d'énergie protégé de l'humidité et des poussières.

Chaque boitier électronique intègre avantageusement des piliers ou plots de soutien 104 afin de renforcer sa solidité, de préférence un plot/cm$^2$ pour résister aux pressions et forces d'impact des mouvements du pied. L'insertion de tels plots permet au boitier de mieux résister au poids d'une personne. De manière avantageuse, ces plots ou encore muret, permettent également de fixer une carte électronique afin de fixer le centre inertiel pour ne pas fausser les mesures. Ainsi, de façon préférée, le boitier électronique 100 selon l'invention comporte au moins deux plots de soutien 104, de façon plus préférée au moins trois plots de soutien 104 et de façon encore plus préférée au moins quatre plots de soutien 104.

En outre, le boitier électronique 100 selon l'invention peut comporter un muret en polymère positionné entre la source d'énergie et la carte électronique de façon à pouvoir augmenter sa robustesse. Un tel muret présente par exemple une hauteur correspondant à la hauteur du boitier, une épaisseur comprise entre 0,5 et 3 mm et une longueur comprise entre 3/5 et 5/5 de la largeur de la source d'énergie.

Avantageusement, le boitier électronique 100 comporte une carte électronique présentant au moins une ouverture 105 permettant le passage d'au moins un plot de soutien 104, de préférence au moins deux ouvertures 105.

En outre, de façon à augmenter encore la robustesse du système, chaque boitier électronique comporte un matériau absorbeur de chocs tel que de la mousse polymère (e.g. polyuréthane, polyéther). Selon un mode de réalisation, le matériau absorbeur de chocs présente une densité comprise entre 20 kg/m$^3$ et 50 kg/m$^3$. Une telle couche de mousse protectrice permet également d'isoler la carte des vibrations et de l'humidité.

De façon préférée, chacun des boitiers (e.g. droit et gauche) présente une forme différente de l'autre boitier. Par exemple, il comporte une saillie n'étant pas présente sous une forme identique sur l'autre boitier. Une telle caractéristique physique permet de différencier les deux boitiers, droit ou gauche, lors de leur intégration dans une chaussure ou une semelle sans inverser le centre inertiel.

Selon un mode de réalisation de l'invention, la carte électronique est insérée dans un compartiment du boitier spécialement conçu pour la recevoir.

Selon un autre mode de réalisation, le boitier électronique 100 est formé par l'encapsulation de ses composants. Par exemple, l'encapsulation peut prendre la forme un revêtement enrobant ou d'une résine (e.g. silicone, époxy, polyuréthane). L'encapsulation de l'intégralité des composants (e.g. plateforme inertielle, module de traitement...) offre une bonne isolation et combine ainsi de bonnes propriétés électriques à une excellente protection mécanique.

En outre, le boitier électronique selon l'invention comporte une plateforme inertielle 110,111,112 configurée pour générer un ensemble de données sur la démarche d'un utilisateur de la paire 10 de semelles.

Au cours de la marche d'un utilisateur, la plateforme inertielle 110 acquière des signaux représentatifs d'un paramètre de mouvement (accélération et/ou vitesse, par exemple vitesse angulaire) du pied selon les axes X, Y, Z. En outre, ces données peuvent ensuite être traitées pour générer au moins un signal d'accélération. La plateforme inertielle est par exemple constituée d'au moins un accéléromètre et un gyroscope. De façon préférée, elle comporte plusieurs accéléromètres et gyroscopes.

Le boitier électronique peut également comporter un ou plusieurs magnétomètres de façon à acquérir trois signaux bruts supplémentaires correspondant aux valeurs de champs magnétiques sur trois dimensions.

Chaque boitier électronique peut comporter par ailleurs d'autres capteurs, notamment un inclinomètre, un baromètre, un capteur de température et un altimètre pour bénéficier d'une précision accrue.

En outre, le boitier électronique selon l'invention comporte un module de traitement de données 120,121,122 configurée pour transformer l'ensemble des données générées grâce à des algorithmes prédéfinis. En effet, le dispositif ou système selon l'invention permet que les données reçues via les capteurs situés dans les semelles intérieures et/ou extérieures sont traitées selon un ou plusieurs algorithmes dans chacun des boîtiers, lesdits boîtiers étant paramétrés en un boîtier Slave, qui reçoit les données de sa semelle/chaussure et les transmet au boîtier Master, dit boîtier Master (boîtier principal) qui reçoit les données du boîtier Slave, les traite en les comparant à ses propres données et génère une information sur la posture de l'utilisateur en général et de ses pieds en particulier, information que le boîtier transmet au terminal en temps réel ou de manière différée.

Le module de traitement 120,121,122 permet d'analyser en 3D la posture, les mouvements, la locomotion, l'équilibre et l'environnement de l'utilisateur, et plus généralement tout ce qui sera qualifié comme étant sa marche, à partir des données recueillies par la plateforme inertielle et les éventuels capteurs complémentaires placés dans la semelle.

Ce module de traitement peut être utilisé pour générer des paramètres biomécaniques de la démarche. Avantageusement, le module de traitement de données 120 est apte à transformer l'ensemble de données en au moins un paramètre biomécanique de la démarche, ledit paramètre biomécanique de la démarche étant de préférence sélectionné parmi : la posture, la pronation, la supination, la force d'impact, la zone d'impact, la longueur des pas, le temps de contact, le temps de vol, la boiterie, la force de propulsion, l'équilibre et plusieurs autres paramètres relatifs à l'utilisateur et décrivant sa démarche, ses postures et ses mouvements.

En outre, la transformation par le module de traitement de données peut avantageusement comprendre la segmentation des données en une pluralité de phases. De préférence, le module de traitement de données est apte à segmenter un pas en au moins quatre phases telles que : la phase d'impact (correspond au moment précis du contact du pied avec le sol), la phase d'appui (se déroule depuis la phase d'impact jusqu'au décollement du talon du sol), la phase de propulsion (débute quand le talon a quitté le sol et se termine quand le premier orteil a quitté le sol) et la phase de vol (débute quand le premier orteil a quitté le sol et se termine quand le talon touche le sol).

De façon plus particulière, le découpage ou la segmentation du pas peut permettre d'identifier les zones principales d'appui de l'utilisateur. Ainsi, le système peut être utilisé pour mesurer la forme du pas pendant la marche ou toute autre activité de l'utilisateur afin de déterminer les malformations éventuelles des pieds et postures de l'utilisateur.

Les informations générées vont ensuite être transmises au second boitier par une émission de signaux qui peuvent être de type Bluetooth.

Lorsqu'un boitier électronique n'est pas en mesure de communiquer en temps réel avec l'autre boitier et/ou avec le terminal, il stocke les informations récoltées et les transmettra en différé lorsque l'échange sera de nouveau possible. Cette transmission en différé des données recueillies est rendue possible grâce à la capacité de stockage dont est doté chacun des boitiers électroniques.

Ainsi, le boitier électronique selon l'invention comporte un module de stockage 130,131,132 de données, configuré pour mémoriser au moins une partie des données transformées et/ou des données générées par le module de traitement. Comme cela a déjà été discuté, le dispositif ou système selon l'invention est tel qu'il permet de fonctionner avec un module de stockage de données de faible capacité. Par exemple, le module de stockage 130,131,132 de données peut avantageusement présenter une capacité de mémoire inférieure à 512 ko, de préférence inférieure à 128 ko, de façon plus préférée inférieure à 32 ko et de façon encore plus préférée inférieure à 16 ko. En particulier, le module de stockage peut correspondre à de la mémoire disponible sur un CPU.

En outre, le boitier électronique selon l'invention comporte des moyens de communications. Ainsi, en particulier, chacun des boîtiers, qu'il soit Slave ou Master, est conçu de manière à pouvoir communiquer indépendamment avec l'autre et/ou directement avec le terminal afin de pouvoir échanger ses propres informations sur la posture/le mouvement/l'activité de son pied, dont il a reçu les données via les différents capteurs de sa semelle intérieure et/ou extérieure de chaussure

De préférence, le boitier électronique selon l'invention comporte un premier moyen de communication 140,141,142 configuré de façon à ce que le boitier électronique 100 d'au moins une des semelles soit apte à transmettre au moins une partie des données transformées à un terminal externe 20. Ces données peuvent être transmises en temps réel ou en différé à un terminal externe 20. Le terminal externe 20 peut par exemple être un système distant tel qu'une tablette, un téléphone mobile (« smartphone » en terminologie anglosaxonne), un ordinateur ou un serveur.

Avantageusement, chaque boitier électronique comporte en outre un second moyen de communication configuré de façon à ce que le boitier électronique 101 d'une première semelle soit apte à communiquer avec le boitier électronique 102 d'une seconde semelle, et en ce qu'au moins un module de traitement de données 121,122 soit configuré pour calculer, de préférence conjointement, des ensembles de données générés à partir des deux semelles 11,12, et plus particulièrement de la plateforme inertielle, composant la paire 10 de semelles. En effet, le calcul de certains paramètres biomécaniques d'intérêt nécessite les données provenant des deux semelles.

Les premiers et deuxièmes moyens de communication peuvent consister en un seul et même moyen.

Les premiers et deuxièmes moyens de communication sont aptes à recevoir et à transmettre les données sur au moins un réseau de communication. De préférence la communication est opérée par l'intermédiaire d'un protocole sans fils tel que wifi, 3G, 4G, et/ou Bluetooth. De préférence le protocole de communication est un protocole BLE ou ANT+. Ces protocoles de communications permettent une consommation faible en énergie.

Avantageusement, du fait de son confinement à l'intérieur d'un boitier placé sous le corps d'une personne, l'antenne doit de préférence être disposée à l'intérieur du boitier sur le côté orienté vers le côté extérieur de la semelle. Ce positionnement de l'antenne est préférable dans la mesure où des tests en laboratoire ont permis d'établir que le signal émis depuis une semelle ou une chaussure est absorbée à 70 % par le corps humain. Cette antenne doit donc être positionnée en périphérie du pied et orientée de telle manière à toujours pouvoir transmettre le signal au terminal externe et/ou au boitier de la seconde semelle. De façon préférée, l'antenne peut être une antenne imprimée sur une carte électronique. Alternativement, l'antenne peut être imprimée sur une face intérieure du boitier et connectée à la carte électronique par câblage. L'antenne peut de préférence être positionnée sur une partie basse par rapport à la carte électronique. Ainsi, la carte électronique vient faire contact avec l'antenne.

En outre, le boitier électronique selon l'invention comporte une source d'énergie 150,151,152. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

En outre, le boitier électronique selon l'invention peut comporter un moyen de connexion filaire 160, de préférence protégé par une languette amovible. Ce moyen de connexion filaire peut être par exemple un port USB ou firewire. Avantageusement, le port USB est également résistant à l'eau ou l'humidité. En outre, le port USB est avantageusement surmonté d'une poutrelle en polymère permettant de lui conférer une plus grande résistance en condition d'utilisation. Ce moyen de connexion filaire peut être utilisé comme évoqué ci-dessus pour recharger la batterie mais également pour échanger des données et par exemple mettre à jour le micrologiciel de la carte électronique portant les différents composants du boitier électronique.

De préférence, la languette amovible ou USB cover, permet de protéger le port USB de corps étrangers. Par exemple, la languette amovible permet de protéger le port USB de l'eau ou de la poussière. Une telle languette peut de préférence être réalisée en polymère de type élastomère ou polyuréthane.

Ces différents composants du boitier électronique sont de préférence agencés sur une carte électronique 170 (ou circuit imprimé). En outre, les différents moyens et modules du boitier électronique 100 sont représentés de façon distincte sur les figures 1 et 2 mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique.

En outre, le système 1 comporte un terminal externe 20 apte à recevoir des données. Le terminal externe 20 est généralement une tablette, un téléphone mobile (« smartphone » en terminologie anglosaxonne), une passerelle, un routeur, un ordinateur ou un serveur. Il peut être apte à transférer ces données à un serveur distant 30. Il est alors par exemple possible d'accéder à ce serveur distant via une interface web.

Avantageusement, une application dédiée est installée sur ce terminal externe afin de traiter les informations transmises par les boitiers et permettre à l'utilisateur d'interagir avec l'invention.

Avantageusement, selon un mode de réalisation de l'invention, les deux boitiers seront préférablement paramétrés en un boitier Principal et un boitier Secondaire. Le boitier Principal va recevoir des données sur la position et/ou l'activité dans laquelle se trouve la semelle Secondaire ; le boitier Principal va traiter ces données et en tirer une information en toutes circonstances (par exemple, lorsque l'utilisateur est en position agenouillée, semelles relevées de manière plus ou moins verticale, chacun des boitiers va constater que sa semelle est dans une position relevée plus ou moins perpendiculairement au sol ; le boitier Principal constatant qu'il est dans la même position que le boitier Secondaire, il pourra en déduire que l'utilisateur est dans une position à genou).

Dès lors, même si les deux semelles sont dans des positions différentes, les deux boitiers continuent à détecter les positions de chaque semelle de manière à enregistrer les changements de postures de l'utilisateur. Cette détection continue des postures de l'utilisateur permet, par comparaison dans le temps, de relever avec précision les évolutions dans la marche, la posture ou l'activité physique, sportive ou professionnelle de celui-ci, de sorte que le dispositif peut également relever toutes éventuelles anomalies, telle une éventuelle boiterie.

Le boitier va donc pouvoir non seulement constater les différentes positions mais également relever des carences ou des anomalies qui apparaissent au niveau de la locomotion, de l'équilibre, ou plus généralement de la marche de l'utilisateur.

Le boitier Principal va analyser et stocker les données recueillies puis transmettre l'information en temps réel ou en différé au terminal. Grâce au module de stockage de chacun des boitiers, l'essentiel des données récoltées va pouvoir être stocké tant que les boitiers ne sont pas reliés à un terminal externe, de sorte qu'il n'y a pas de perte de données ni d'information concernant l'utilisateur.

Selon un mode de réalisation de l'invention, le boitier Secondaire peut également transmettre les données recueillies à un terminal extérieur.

Outre l'analyse de la marche et de l'activité de son utilisateur, cette invention a pour objet d'identifier des carences ou des problèmes liés à la posture des pieds. Cette posture est révélatrice des problèmes mécaniques, physiques ou physiologiques de l'utilisateur, notamment lorsqu'il marche.

De la posture du pied et de son mouvement, toutes sortes d'informations sont mesurées par la carte électronique situé dans le boitier. L'utilisateur peut avoir une démarche qui n'est pas appropriée, avoir des pas irréguliers, un pattern de marche inadéquat, une pronation ou une supination non décelés...

Ce boitier va pouvoir analyser les données relatives aux postures de chacun des deux pieds déceler plusieurs anomalies, perceptibles par la simple analyse de la locomotion, le pattern ou l'équilibre de l'utilisateur, ou très généralement la marche ou l'activité de celui-ci.

Selon un autre aspect, l'invention porte sur un procédé 200 de quantification de la démarche d'un utilisateur, de préférence mis en œuvre au sein d'un système selon l'invention.

Les inventeurs ont testé plusieurs procédés afin de quantifier la posture et la démarche d'un utilisateur sans altérer son confort et tout en minimisant la consommation en énergie du procédé. La plupart des procédés testés ont une consommation en énergie trop élevée ce qui se traduit par un besoin en énergie important et donc des composants plus volumineux, ce qui altère le confort de l'utilisateur. En effet, les inventeurs ont lors d'essais des procédés de traitement de données de plateforme inertielle habituels, mis en exergue une consommation trop importante de l'énergie stockée dans le boitier de même que de la mémoire. En effet, avec les procédés de traitement classiques, faisant par exemple appel à des analyses de motif sur fenêtre glissante à partir des données brutes des deux pieds, la consommation en énergie et en mémoire réduisait l'autonomie du dispositif à quelques dizaines de minutes. Cela, même lorsque les calculs étaient mis en œuvre sur des téléphones, des montres connectés ou encore des serveurs distants.

Les inventeurs ont donc mis au point un nouveau procédé de quantification de la posture et de la démarche d'un utilisateur pouvant être mis en œuvre au niveau de boitiers intégrés dans des semelles. Ce procédé peut en particulier donner des résultats pertinents sur la démarche malgré une mémoire de taille réduite (par exemple comprise entre 128 ko et 512 ko) alors que les procédés antérieurs auraient nécessité beaucoup plus de mémoire ou des sources d'énergie beaucoup plus importante.

Pour cela les inventeurs ont développé un procédé 200 de quantification de la démarche (i.e. de la posture et de la démarche) d'un utilisateur susceptible d'être mis en œuvre par un des systèmes de l'invention et particulièrement adapté pour une mise en œuvre au sein d'un système de quantification de la posture et de la démarche d'un utilisateur comportant un premier boitier 101 intégré dans une première semelle et un second boitier 102 intégré dans une seconde semelle d'une même paire de semelle, le procédé comprenant :

- Une étape de génération 230 de données brutes, par des plateformes inertielles contenues dans le premier et le second boitier, lesdites données brutes étant générée pour une période de temps et étant fonction de la démarche d'un utilisateur,

- Une étape de prétraitement 240 des données brutes, par les modules de traitements de données contenus dans le premier et le second boitier, comprenant la comparaison des données brutes à des motifs prédéterminés et la génération de valeurs de similarités des données brutes avec les motifs prédéterminés,

- Une étape de communication 250 entre le premier et le second boitier, comprenant la transmission, par un module de communication, des valeurs de similarités (des données brutes avec les motifs prédéterminés) du second boitier au premier boitier,

- Une étape de sélection 260, par le premier boitier, d'un motif prédéterminé représentatif des données brutes à partir des valeurs de similarités (des données brutes avec des motifs prédéterminés) du premier boitier et celles du second boitier,

- Une étape de mémorisation 300 de la nouvelle valeur de paramètre de démarche avancé par le premier boitier,

Le procédé selon l'invention comprend une étape préalable d'apprentissage 210, comprenant la définition d'une pluralité de motifs prédéterminés de démarche d'un utilisateur. Ainsi, la répétition de mouvements, postures et démarche d'un utilisateur sur une durée déterminée est enregistrée et classée selon une pluralité de motifs par exemple de type statique ou dynamique. Ainsi un certain motif dynamique représente un mouvement d'un utilisateur tel qu'un « pas » et un motif statique représente une posture de type « à genou » d'un utilisateur. En particulier, les motifs prédéterminés peuvent avoir été normalisés en fonction d'une période de temps correspondant à un événement, l'événement pouvant par exemple être un pas. Dans ce cas, le pas peut être identifié par des méthodes classiques d'étude du mouvement du pas comme cela par exemple la méthode Pan-Tompkins ou la détection de maxima. Ceci afin d'obtenir des motifs représentatifs d'un utilisateur et pouvant varier selon l'utilisateur ou le domaine d'application.

Le procédé selon l'invention comprend également un étape préalable de calibration 220 par un module de calibration, entre un premier et un second boitier comprenant l'émission d'un signal par le premier boitier et la réception du signal émis par le second boitier afin de calibrer un moyen de mesure de temps de préférence une horloge. Un tel calibrage permet au premier et au second boitier de détecter et collecter des données de posture ou de démarche d'un utilisateur sur une même fenêtre temporelle. En effet, les données collectées par le premier et le second boitier sont analysées deux à deux. Ainsi, les données collectées seront analysées en parallèle ce qui permet d'éviter tout décalage entre les données et toutes erreurs d'analyse. De façon préférée, le calibrage entre les deux boitiers est réalisé de façon instantanée et en temps réel.

Avantageusement, si un des boitiers venait à se déconnecter ou perdre la synchronisation dans le temps par rapport à l'autre boitier, le procédé comprend une étape de synchronisation 280 des boitiers. Ainsi, un signal de recherche est envoyé par le boitier connecté, le boitier déconnecté reçoit le signal de recherche et se synchronise sur le boitier connecté. En outre, suite à la synchronisation, les données collectées par le boitier déconnecté sont récupérées.

Le procédé selon l'invention possède une étape de génération 230 de données brutes, par des plateformes inertielles contenues dans le premier et le second boitier, lesdites données brutes étant générées pour une période de temps et étant fonction de la démarche d'un utilisateur. Les données brutes sont relatives à la posture, l'activité ou la démarche d'un utilisateur. La plateforme inertielle du premier et du second boitier génèrent les données brutes via différents capteurs contenus dans les boitiers. Les données brutes peuvent ainsi être issues du gyroscope ou de l'accéléromètre contenu dans chaque boitier. En outre, selon un mode de réalisation avantageux, les données brutes sont collectées par les deux boitiers sur une durée prédéterminée pouvant s'étaler de la milliseconde à la seconde. De façon préférée, les données brutes générées écrasent des données brutes précédemment générées. Ainsi, les données brutes ne sont pas sauvegardées à long terme c'est-à-dire sur une durée supérieure à 30 secondes, de préférence supérieure à 15 secondes, de façon plus préférée à 5 secondes. Ceci permet à la fois une diminution de la consommation en énergie et l'utilisation de composants compacts ce qui se traduit par un meilleur confort pour l'utilisateur.

Le procédé selon l'invention comprend une étape de prétraitement 240 des données brutes, par les modules de traitement de données contenus dans le premier et le second boitier, comprenant la comparaison des données brutes à des motifs prédéterminés et la génération 241 de valeurs de similarités des données brutes avec les motifs prédéterminés. Avantageusement, l'étape de prétraitement comprend la comparaison par le premier et le second boitier des données brutes générées par rapport à la pluralité de motifs prédéterminés, et la génération de valeurs de similarités des données brutes avec les motifs prédéterminés. Le prétraitement comprend le test de l'ensemble de la pluralité des motifs sur chaque ensemble de données brutes générées à la fois sur le premier boitier et sur le second boitier. Ainsi, grâce à ce prétraitement il est possible de catégoriser les données brutes selon des catégories prédéterminées. Il existe différent type de catégories telles que le pas, la montée d'une marche, la descente d'une marche, une foulée, un saut, un temps de vol, un plat, un statisme, un piétinement, un agenouillement... Le nombre de catégorie et le type de catégorie sont fonction de l'application ou de la destination d'utilisation. Les données brutes générées sont alors comparées aux motifs prédéterminés. Ainsi, il est possible de modifier, ajouter, supprimer les différents type de catégories. L'ensemble de la pluralité des motifs sont testés en même temps sur les données brutes. En particulier, sur une période de temps donnée, les données brutes est prétraitée afin de déterminer une pluralité de point qui est comparée à chaque motif de la pluralité de motifs ce qui permet de générer une valeur de similarité pour chacun des motifs. Ceci permet de réduire l'espace mémoire utilisé, le temps de prétraitement des données brutes et donc de minimiser la consommation en énergie. En effet, l'absence de prétraitement répétitif, de chargement à des temps différents sur chaque donnée des différents motifs permet de réduire la consommation en espace mémoire, en énergie et de temps machine. Ceci permet également l'utilisation de composants compact offrant un meilleur confort.

Une fois les données brutes prétraitées et comparées aux motifs prédéterminés, des valeurs de similarités sont générées. Une valeur de similarité est générée pour l'association de chaque donnée brute à chaque type de motifs prédéterminés. La valeur de similarité est calculée par la plateforme inertielle du premier et du second boitier. Avantageusement, les données étant synchronisées, une valeur de similarité est calculée pour une même période de temps et pour chacun des motifs deux fois, une première fois par le premier boitier et une seconde fois par le second boitier. Ainsi il est possible de quantifier la posture ou la démarche d'un utilisateur de façon plus fiable et dont la précision est améliorée.

De préférence, l'étape de prétraitement peut comporter une sous étape de calcul 242 de valeurs provisoires de paramètres de démarche par le premier et le second boitier. Cette étape de calcul comprenant notamment la mise en œuvre d'une pluralité de fonction de calcul, ou règles de calcul, de façon à générer une pluralité de valeurs provisoires à partir des données brutes collectées, par le premier ou le second boitier. Ces paramètres de démarches peuvent par exemple être relatifs à la longueur de la foulée, au temps de vol, à la force d'impact, à la position du pied lors de la phase ascendante ou descendante ou encore à tout autre paramètre relatif à la démarche, l'activité ou la posture d'un utilisateur tel que la pronation ou la supination. En particulier, ces valeurs provisoires de paramètres de démarches sont calculées pour chaque ensemble de données brutes générées sur une période de temps et par chaque boitier. Ces valeurs provisoires de paramètres de démarches permettent d'apporter plus de précision et une meilleure compréhension sur la démarche, l'activité ou la posture d'un utilisateur.

Le procédé selon l'invention comprend en outre, une étape de communication 250 entre le premier et le second boitier, comprenant la transmission, par un module de communication, des valeurs de similarités des données brutes avec les motifs prédéterminés du second boitier au premier boitier. Cette étape de communication entre le premier et le second boitier peut être réalisée selon une fréquence temporelle prédéfinie, par exemple la transmission peut être réalisée toutes les secondes, ou toutes les deux secondes ou pour tout autre fréquence temporelle prédéfinie. Cette étape permet de regrouper l'ensemble des données et particulièrement des valeurs de similarités sur un seul boitier, de façon préférée sur le premier boitier. Pour rappel, de façon préférée chaque motif prédéterminé est donc associé à deux valeurs de similarité l'une provenant du prétraitement des données brutes du premier boitier et l'autre de celles du second boitier. En outre, cette étape de communication peut comporter la transmission des valeurs provisoires de paramètres de démarches d'un second boitier à un premier boitier, ou vis versa.

Le procédé comprend une étape de sélection 260, par le premier boitier, d'une catégorie de mouvement représentative des données brutes à partir des valeurs de similarités du premier boitier et celles du second boitier. Ainsi, les valeurs de similarités permettent au premier boitier d'attribuer aux données brutes générées par les deux boitiers une catégorie de mouvement par exemple à partir d'une liste de catégorie de mouvements prédéterminées. Particulièrement, cette étape comprend la sélection d'une catégorie de mouvement si le second boitier a transmis au premier boitier un motif prédéterminé correspondant avec une valeur de similarité générée supérieure à un pourcentage de fiabilité prédéterminé et mémorisé pour chaque motif prédéterminé. Ceci permet de prendre en compte uniquement des motifs prédéterminé fiables avant de quantifier la posture et de la démarche d'un utilisateur.

Le procédé comprend également une étape de traitement 270 des valeurs provisoires de paramètres de démarche du second boitier et du premier boitier de façon à sélectionner des valeurs provisoires de paramètres de démarche à conserver. En outre, cette étape peut être suivie d'une étape de génération 290 d'une nouvelle valeur de paramètre de démarche par le premier boitier en fonction d'une catégorie de mouvement représentative sélectionnée, de valeurs provisoires de paramètres de démarche sélectionnées, d'une valeur de paramètre de démarche préalablement mémorisée et éventuellement d'un seuil prédéterminé. La valeur de paramètre de démarche préalablement mémorisée est définie pour chaque paramètre de démarche. Ainsi, il est possible de quantifier la démarche et la posture d'un utilisateur de façon fiable et précise, tout en assurant une consommation en énergie réduite et un confort optimal.

En outre, le procédé selon l'invention comporte également une étape de comparaison des valeurs provisoires de paramètres de démarche du second boitier de façon à générer une valeur consolidée de paramètres de démarche pour la période de temps. De préférence, le procédé peut également comporter une étape de transmission de la nouvelle valeur de paramètre de démarche avancé des données mémorisées à un terminal externe. Cette transmission se fait de préférence ponctuellement. La fréquence de transmission donc peut être supérieure à 100 ms, de façon préférée supérieure à 1 seconde. Par exemple, la transmission se fait toutes les 10 secondes. Le terminal externe peut correspondre à un terminal utilisateur est comprendre un ordinateur, une tablette numérique, un téléphone portable, ou plus généralement tout dispositif permettant à un utilisateur de communiquer avec le terminal.

Le procédé comprend une étape de mémorisation 300 de la nouvelle valeur de paramètre de démarche avancé par le premier boitier. Ainsi par opposition aux données brutes et/ou prétraitées qui sont sauvegardés sur une durée courte par exemple sur une mémoire cache, la nouvelle valeur de paramètre de démarche avancé est mémorisée à plus long terme, par exemple sur une mémoire.

## Revendications

1. Système (1) d'analyse et de quantification de la posture et de la démarche d'un utilisateur, ledit système comportant deux boitiers électroniques (100, 101, 102), aptes à être intégrés dans une paire (10) de semelles, un premier boitier étant apte à être intégré dans une première semelle et un second boitier étant apte à être intégré dans une seconde semelle, chaque boitier comprenant :

   - une plateforme inertielle (110, 111, 112) configurée pour générer un ensemble de données sur la posture, l'activité ou la démarche d'un utilisateur de la paire (10) de semelles ;
   - un module de traitement de données (120, 121, 122) configuré pour prétraiter l'ensemble de données recueillies en fonction d'algorithmes prédéfinis et générer une information sur la posture, l'activité ou la démarche d'un utilisateur de la paire de semelles ;
   - un module de stockage de données (130, 131, 132) configuré pour mémoriser l'information générée par le module de traitement ;
   - un moyen de communication (140, 141, 142) configuré de façon à ce qu'un boitier électronique d'au moins une des semelles soit apte à transmettre l'information générée sur l'utilisateur à un terminal externe (20) et/ou à l'autre boitier de l'autre semelle ;
   - une source d'énergie (150),

   ledit système étant **caractérisé en ce que** lesdits boitiers sont configurés de telle sorte que des données brutes générées par la plateforme inertielle (111) d'un premier boitier subissent une première étape de traitement par le module de traitement de données (120) du premier boitier (101) puis que les données traitées soient transférées au second boitier (102) où elles seront à nouveau traitées par le module de traitement de données du second boitier.

2. Système selon la revendication 1, **caractérisé en ce que** les modules de traitement de données contenus dans le premier et le second boitier sont configurés pour mettre en oeuvre une étape de prétraitement (240) des données brutes comprenant la comparaison des données brutes à des motifs prédéterminés et la génération (241) de valeurs de similarités des données brutes avec les motifs prédéterminés ; et **en ce que** le deuxième boitier est configuré pour sélectionner (260) une catégorie de mouvement représentative des données brutes à partir des valeurs de similarités du premier boitier et celles du second boitier.

3. Système selon la revendication 1, **caractérisé en ce que** les modules de traitements de données contenus dans le premier et le second boitier, sont configurés pour comparer des données brutes générées par la plateforme inertielle du boitier respectif à des motifs prédéterminés et pour générer des valeurs de similarités des données brutes avec les motifs prédéterminés, lesdits motifs prédéterminés correspondants à une catégorie de mouvement prédéterminée, l'un des modules de traitements étant en outre configuré pour sélectionner une catégorie de mouvement représentative des données brutes générées sur une période de temps, à partir des valeurs de similarités du premier boitier et celles du second boitier.

4. Système selon l'une quelconque des revendications 1 à 3, **caractérisé en ce que** la plateforme inertielle (110, 111, 112) comporte au moins un accéléromètre et au moins un gyroscope, et peut être complétée par d'autres capteurs, notamment un magnétomètre, un baromètre et un altimètre.

5. Système selon l'une quelconque des revendications précédentes, **caractérisé en ce que** chacun des boitiers (100, 101, 102), outre la carte électronique (170) et une source d'énergie (150), est également être connecté à un GPS et/ou à tous types de capteurs, notamment des capteurs physiologiques, capteurs de pression, de température ou tout système de climatisation placés dans la semelle.

6. Système selon l'une quelconque des revendications précédentes, **caractérisé en ce que** chaque boitier électronique comporte au moins un plot de soutien (104), de préférence au moins deux plots de soutien (104).

7. Système selon l'une quelconque des revendications précédentes, **caractérisé en ce que** chaque boitier électronique (100, 101, 102) pèse moins de 20 grammes.

8. Système selon l'une quelconque des revendications précédentes, **caractérisé en ce que** chaque boitier électronique (100, 101, 102) présente une épaisseur inférieure ou égale à 7 mm.

9. Système selon l'une quelconque des revendications précédentes, **caractérisé en ce que** chaque boitier électronique (100, 101, 102) présente une surface sur sa face la plus grande inférieure ou égale à 10 $cm^2$.

10. Système selon l'une quelconque des revendications précédentes, **caractérisé en ce que** chaque boitier électronique (100, 101, 102) comporte une enveloppe extérieure (103), ladite enveloppe extérieure étant essentiellement constituée d'un matériau thermoplastique lui permettant de résister à de fortes contraintes mécaniques, correspondant au minimum à 100 000 impacts de 1000 N à une fréquence de 1Hz ou 100 000 impacts de 3000 N à une fréquence de 2,6 Hz, lesdits boitiers étant par ailleurs résistant à la poussière et l'humidité à un niveau au minimum IP56.

11. Système selon l'une quelconque des revendications précédentes, **caractérisé en ce que** la source d'énergie (150) du boitier électronique (100, 101, 102) est une batterie rechargeable, dont la recharge peut être réalisée selon différentes technologies :

par chargeur, avec un connecteur affleurant au niveau de la semelle ;
avec un dispositif de recharge mécanique intégré à la semelle, comme par exemple un dispositif piézoélectrique apte à fournir une énergie électrique à partir de la marche ;
avec un dispositif sans contact, par exemple par induction ; ou avec un dispositif photovoltaïque.

12. Procédé (200) de quantification de la posture et de la démarche d'un utilisateur mis en oeuvre au sein d'un système comportant deux boitiers électroniques (100, 101, 102) intégrés dans une paire (10) de semelles, un premier boitier (101) étant intégré dans une première semelle et un second boitier (102) étant intégré dans une seconde semelle d'une même paire de semelle, chaque boitier comprenant : une plateforme inertielle (110, 111, 112), un module de traitement de données (120, 121, 122), un module de stockage de données (130, 131, 132) configuré pour mémoriser l'information générée par le module de traitement, un moyen de communication (140, 141, 142) et une source d'énergie (150), ledit procédé comprenant :

- Une étape de génération (230) de données brutes, par des plateformes inertielles contenues dans le premier et le second boitier, lesdites données brutes étant générées pour une période de temps et étant fonction de la démarche d'un utilisateur,

- Une étape de prétraitement (240) des données brutes, par les modules de traitements de données contenus dans le premier et le second boitier, comprenant la comparaison des données brutes à des motifs prédéterminés et la génération de valeurs de similarités des données brutes avec les motifs prédéterminés, lesdits motifs prédéterminés correspondants à une catégorie de mouvement prédéterminée,

- Une étape de communication (250) entre le premier et le second boitier, comprenant la transmission, par un module de communication, des valeurs de similarités du second boitier au premier boitier, et

- Une étape de sélection (260), par le premier boitier, d'une catégorie de mouvement représentative des données brutes générées sur la période de temps, à partir des valeurs de similarités du premier boitier et celles du second boitier.

13. Procédé (200) de quantification de la posture et de la démarche d'un utilisateur selon la revendication précédente **caractérisé en ce qu'**il comprend en outre une étape d'incrémentation d'un compteur associé à la catégorie de mouvement sélectionnée et une mémorisation de la nouvelle valeur dudit compteur.

14. Procédé (200) de quantification de la posture et de la démarche d'un utilisateur selon l'une des revendications 12 ou 13, **caractérisé en ce que** l'étape de prétraitement (240) comporte une sous étape de calcul (242) de valeurs provisoires de paramètres de démarche par le premier et le second boitier.

15. Procédé (200) de quantification de la posture et de la démarche d'un utilisateur selon la revendication 14, **caractérisé en ce qu'**il comprend une étape de traitement (270) des valeurs provisoires de paramètres de démarche du second boitier et du premier boitier de façon à sélectionner des valeurs provisoires de paramètres de démarche à conserver.

16. Procédé (200) de quantification de la posture et de la démarche d'un utilisateur selon l'une quelconque des revendications 12 à 15, **caractérisé en ce qu'**il comprend une étape préalable d'apprentissage (210) comprenant une définition d'une pluralité de motifs prédéterminés de démarche d'un utilisateur.

**Patentansprüche**

1. System (1) zur Analyse und Quantifizierung der Körperhaltung und des Gangs eines Benutzers, wobei das System zwei elektronische Boxen (100, 101, 102) umfasst, die in ein Paar (10) Sohlen integriert werden können, wobei eine erste Box in eine erste Sohle integriert werden kann und eine zweite Box in eine zweite Sohle integriert werden kann, wobei jede Box umfasst:

- eine Trägheitsplattform (110, 111, 112), die dafür konfiguriert ist, einen Datensatz über die Körperhaltung, die Aktivität oder den Gang eines Benutzers des Paares (10) Sohlen zu erzeugen;
- ein Datenverarbeitungsmodul (120, 121, 122), das dafür konfiguriert ist, den erfassten Datensatz gemäß vordefinierter Algorithmen vorzuverarbeiten und Informationen über die Körperhaltung, die Aktivität oder den Gang eines Benutzers des Sohlenpaares zu erzeugen;
- ein Datenspeichermodul (130, 131, 132), das dafür konfiguriert ist, die von dem Verarbeitungsmodul erzeugten Informationen zu speichern;
- Kommunikationsmittel (140, 141, 142), das so konfiguriert ist, dass eine elektronische Box von mindestens einer der Sohlen geeignet ist, die über den Benutzer erzeugten Informationen an ein externes Terminal (20) und/oder an die andere Box der anderen Sohle zu übermitteln;
- eine Energiequelle (150),

wobei das System **dadurch gekennzeichnet ist, dass** die Boxen so konfiguriert sind, dass die von der Trägheitsplattform (111) einer ersten Box erzeugten Rohdaten einem ersten Verarbeitungsschritt durch das Datenverarbeitungsmodul (120) der ersten Box (101) unterzogen werden und die verarbeiteten Daten dann an die zweite Box (102) weitergeleitet werden, wo sie von dem Datenverarbeitungsmodul der zweiten Box erneut verarbeitet werden.

2. System nach Anspruch 1, **dadurch gekennzeichnet, dass** die in der ersten und der zweiten Box enthaltenen Datenverarbeitungsmodule dafür konfiguriert sind, einen Vorverarbeitungsschritt (240) der Rohdaten durchzuführen, der den Vergleich der Rohdaten mit vorbestimmten Mustern und die Erzeugung (241) von Ähnlichkeitswerten der Rohdaten mit den vorbestimmten Mustern umfasst; und dass die zweite Box dafür konfiguriert ist, ausgehend von den Ähnlichkeitswerten der ersten Box und denjenigen der zweiten Box, eine Bewegungskategorie auszuwählen (260), die die Rohdaten repräsentiert.

3. System nach Anspruch 1, **dadurch gekennzeich-**

**net, dass** die in der ersten und der zweiten Box enthaltenen Datenverarbeitungsmodule dafür konfiguriert sind, Rohdaten, die von der Trägheitsplattform der jeweiligen Box erzeugt werden, mit vorbestimmten Mustern zu vergleichen und Ähnlichkeitswerte der Rohdaten mit den vorbestimmten Mustern zu erzeugen, wobei die vorbestimmten Muster einer vorbestimmten Bewegungskategorie entsprechen, wobei eines der Verarbeitungsmodule außerdem dafür konfiguriert ist, ausgehend von den Ähnlichkeitswerten der ersten Box und denjenigen der zweiten Box, eine Bewegungskategorie auszuwählen, die die über einen Zeitraum erzeugten Rohdaten repräsentiert.

4. System nach einem der Ansprüche 1 bis 3, **dadurch gekennzeichnet, dass** die Trägheitsplattform (110, 111, 112) mindestens einen Beschleunigungsmesser und mindestens ein Gyroskop umfasst und durch andere Sensoren, insbesondere ein Magnetometer, ein Barometer und einen Höhenmesser, ergänzt werden kann.

5. System nach einem der vorstehenden Ansprüche, **dadurch gekennzeichnet, dass** jede der Boxen (100, 101, 102) außer an die elektronische Karte (170) und eine Energiequelle (150) auch an ein GPS und/oder an alle Arten von in der Sohle platzierten Sensoren, insbesondere physiologische Sensoren, Drucksensoren, Temperatursensoren oder jegliches Klimatisierungssystem angeschlossen ist.

6. System nach einem der vorstehenden Ansprüche, **dadurch gekennzeichnet, dass** jede elektronische Box mindestens ein Stützkissen (104), vorzugsweise mindestens zwei Stützkissen (104), aufweist.

7. System nach einem der vorstehenden Ansprüche, **dadurch gekennzeichnet, dass** jede elektronische Box (100, 101, 102) weniger als 20 Gramm wiegt.

8. System nach einem der vorstehenden Ansprüche, **dadurch gekennzeichnet, dass** jede elektronische Box (100, 101, 102) eine Dicke von weniger als oder gleich 7 mm aufweist.

9. System nach einem der vorstehenden Ansprüche, **dadurch gekennzeichnet, dass** jede elektronische Box (100, 101, 102) an ihrer größten Seite eine Fläche von weniger als oder gleich 10 cm$^2$ aufweist.

10. System nach einem der vorstehenden Ansprüche, **dadurch gekennzeichnet, dass** jede elektronische Box (100, 101, 102) eine äußere Hülle (103) aufweist, wobei die äußere Hülle im Wesentlichen aus einem thermoplastischen Material besteht, das es ihr ermöglicht, starken mechanischen Belastungen standzuhalten, die mindestens 100.000 Stößen von 1.000 N bei einer Frequenz von 1 Hz oder 100.000 Stößen von 3.000 N bei einer Frequenz von 2,6 Hz entsprechen, wobei die Boxen außerdem mit einem Schutzgrad von mindestens IP56 staub- und feuchtigkeitsbeständig sind.

11. System nach einem der vorstehenden Ansprüche, **dadurch gekennzeichnet, dass** die Energiequelle (150) der elektronischen Box (100, 101, 102) ein wiederaufladbarer Akku ist, der mit verschiedenen Technologien wieder aufgeladen werden kann:

     per Ladegerät, mit einem mit der Sohle bündigen Anschluss;
     mit einer in die Sohle integrierten mechanischen Aufladevorrichtung, wie zum Beispiel einer piezoelektrischen Vorrichtung, die in der Lage ist, elektrische Energie aus dem Gehen zu liefern;
     mit einer berührungslosen Vorrichtung, zum Beispiel durch Induktion;
     oder mit einer Photovoltaikvorrichtung.

12. Verfahren (200) zur Quantifizierung der Körperhaltung und des Gangs eines Benutzers, das mit einem System mit zwei elektronischen Boxen (100, 101, 102) implementiert wird, welche in ein Paar (10) Sohlen integriert sind, wobei eine erste Box (101) in eine erste Sohle und eine zweite Box (102) in eine zweite Sohle desselben Sohlenpaars integriert ist, wobei jede Box umfasst:

     eine Trägheitsplattform (110, 111, 112), ein Datenverarbeitungsmodul (120, 121, 122), ein Datenspeichermodul (130, 131, 132), das dafür konfiguriert ist, die von dem Verarbeitungsmodul erzeugten Informationen zu speichern, Kommunikationsmittel (140, 141, 142) und eine Energiequelle (150),
     wobei das Verfahren umfasst:

         - einen Schritt der Erzeugung (230) von Rohdaten durch Trägheitsplattformen, die in der ersten und der zweiten Box enthalten sind, wobei die Rohdaten für einen bestimmten Zeitraum erzeugt werden und vom Gang eines Benutzers abhängen;
         - einen Schritt der Vorverarbeitung (240) der Rohdaten durch die in der ersten und der zweiten Box enthaltenen Datenverarbeitungsmodule, umfassend den Vergleich der Rohdaten mit vorbestimmten Mustern und die Erzeugung von Ähnlichkeitswerten der Rohdaten mit den vorbestimmten Mustern, wobei die vorbestimmten Muster einer vorbestimmten Bewegungskategorie entsprechen,
         - einen Schritt der Kommunikation (250) zwischen der ersten und der zweiten Box, umfassend die Übermittlung der Ähnlichkeitswerte von der zweiten zu der ersten Box durch ein Kommunikationsmodul, und
- einen Schritt der Auswahl (260), durch die erste Box, einer Bewegungskategorie, die die in dem Zeitraum erzeugten Rohdaten repräsentiert, ausgehend von den Ähnlichkeitswerten der ersten Box und denjenigen der zweiten Box.

**13.** Verfahren (200) zur Quantifizierung der Körperhaltung und des Gangs eines Benutzers nach dem vorstehenden Anspruch, **dadurch gekennzeichnet, dass** es zusätzlich einen Schritt der Inkrementierung eines Zählers umfasst, der der ausgewählten Bewegungskategorie zugeordnet ist, und eine Speicherung des neuen Wertes dieses Zählers.

**14.** Verfahren (200) zur Quantifizierung der Körperhaltung und des Gangs eines Benutzers nach einem der Ansprüche 12 oder 13, **dadurch gekennzeichnet, dass** der Schritt der Vorverarbeitung (240) einen Teilschritt der Berechnung (242) von vorläufigen Gangparameterwerten durch die erste und die zweite Box umfasst.

**15.** Verfahren (200) zur Quantifizierung der Körperhaltung und des Gangs eines Benutzers nach Anspruch 14, **dadurch gekennzeichnet, dass** es einen Schritt der Verarbeitung (270) der vorläufigen Gangparameterwerte der zweiten und der ersten Box umfasst, um vorläufige Gangparameterwerte auszuwählen, die beibehalten werden sollen.

**16.** Verfahren (200) zur Quantifizierung der Körperhaltung und des Gangs eines Benutzers nach einem der Ansprüche 12 bis 15, **dadurch gekennzeichnet, dass** es einen vorherigen Schritt des Lernens (210) umfasst, der eine Definition einer Vielzahl von vorbestimmten Gangmustern eines Benutzers umfasst.

**Claims**

**1.** A system (1) of analyzing and quantifying a user's posture and gait, said system including two electronic boxes (100, 101, 102), adapted to be integrated into a pair (10) of soles, a first box being adapted to be integrated into a first sole and a second box being adapted to be integrated into a second sole, each box comprising:

- an inertial platform (110, 111, 112) configured to generate a set of data on the posture, activity or gait of a user of the pair (10) of soles;
- a data processing module (120, 121, 122) configured to pre-process the collected data set according to predefined algorithms and generate information on the posture, activity or gait of a user of the pair of soles;
- a data storage module (130, 131, 132) configured to store the information generated by the processing module;
- a means of communication (140, 141, 142) configured so that an electronic box of at least one of the soles is adapted to transmit the information generated on the user to an external terminal (20) and/or to the other box of the other sole;
- a power source (150),

said system being **characterized in that** the boxes are configured such that the raw data generated by the inertial platform (111) of a first box undergoes a first processing step by the data processing module (120) of the first box (101), and then the processed data is transferred to the second box (102) where it is further processed by the data processing module of the second box.

**2.** The system according to claim 1, **characterized in that** the data processing modules contained in the first and second boxes are configured to implement a step of preprocessing (240) of the raw data, comprising the comparison of the raw data with predetermined patterns and the generation (241) of values of similarities of the raw data with the predetermined patterns; and **in that** the second box is configured to select (260) a movement category representative of the raw data from the similarity values of the first box and those of the second box.

**3.** The system according to claim 1, **characterized in that** the data processing modules contained in the first and second box are configured to compare raw data generated by the inertial platform of the respective box with predetermined patterns and to generate similarity values of the raw data with the predetermined patterns, said predetermined patterns corresponding to a predetermined movement category, one of the processing modules being further configured to select a movement category representative of the raw data generated over a period of time, from the similarity values of the first box and those of the second box.

**4.** The system according to any one of claims 1 to 3, **characterized in that** the inertial platform (110, 111, 112) includes at least one accelerometer and at least one gyroscope, and can be added further sensors, in particular a magnetometer, a barometer and an altimeter.

**5.** The system according to any one of the preceding claims, **characterized in that** each of the boxes (100, 101, 102), in addition to the electronic board (170) and a power source (150), can also be connected to a GPS and/or to all types of sensors, in particular physiological sensors, pressure sensors, temperature sensors or any air-conditioning system placed in the sole.

6. The system according to any one of the preceding claims, **characterized in that** each electronic box includes at least one support pad (104), preferably at least two support pads (104).

7. The system according to any one of the preceding claims, **characterized in that** each electronic box (100, 101, 102) weighs less than 20 grams.

8. The system according to any one of the preceding claims, **characterized in that** each electronic box (100, 101, 102) has a thickness less than or equal to 7 mm.

9. The system according to any one of the preceding claims, **characterized in that** each electronic box (100, 101, 102) has a surface area on its largest side less than or equal to 10 cm$^2$.

10. The system according to any one of the preceding claims, **characterized in that** each electronic box (100, 101, 102) includes an outer casing (103), said outer casing being essentially made of a thermoplastic material enabling it to withstand high mechanical stresses, corresponding to at least 100,000 impacts of 1000 N at a frequency of 1 Hz or 100,000 impacts of 3000 N at a frequency of 2.6 Hz, said boxes being, furthermore, resistant to dust and humidity at a level of at least IP56.

11. The system according to any one of the preceding claims, **characterized in that** the power source (150) of the electronic box (100, 101, 102) can be a rechargeable battery, which can be recharged using different technologies:

   by a charger, with a connector flush with the sole;
   with a mechanical recharging device integrated into the sole, such as a piezoelectric device capable of supplying electrical energy from walking;
   with a non-contact device, for example by induction; or
   with a photovoltaic device.

12. A method (200) of quantifying a user's posture and gait implemented within a system including two electronic boxes (100, 101, 102) integrated into a pair (10) of soles, a first box (101) being integrated into a first sole and a second box (102) being integrated into a second sole of a same pair of soles, each box comprising: an inertial platform (110, 111, 112), a data processing module (120, 121, 122), a data storage module (130, 131, 132) configured to store the information generated by the processing module, a means of communication (140, 141, 142) and a power source (150), said method comprising:

   - A step of generating (230) raw data, by inertial platforms contained in the first and second box, said raw data being generated for a period of time and being a function of a user's gait,
   - A step of pre-processing (240) the raw data, by the data processing modules contained in the first and second box, comprising comparing the raw data with predetermined patterns and generating similarity values of the raw data with the predetermined patterns, said predetermined patterns corresponding to a predetermined category of movement,
   - A step of communicating (250) between the first and second box, comprising transmitting, by a communication module, similarity values from the second box to the first box, and
   - A step of selecting (260), by the first box, a movement category representative of the raw data generated over the period of time, from the similarity values of the first box and those of the second box.

13. The method (200) of quantifying a user's posture and gait according to the preceding claim, **characterized in that** it further comprises a step of incrementing a counter associated with the selected category of movement and storing the new value of said counter.

14. The method (200) of quantifying a user's posture and gait according to one of claims 12 or 13, **characterized in that** the pre-processing step (240) includes a sub-step of calculating (242) preliminary values of gait parameters by the first and second box.

15. The method (200) of quantifying a user's posture and gait according to claim 14, **characterized in that** it comprises a step of processing (270) the preliminary values of gait parameters of the second box and of the first box so as to select preliminary values of gait parameters to be retained.

16. The method (200) of quantifying a user's posture and gait according to any one of claims 12 to 15, **characterized in that** it comprises a prior learning step (210) comprising defining a plurality of predetermined patterns of a user's gait.

**FIG. 1**

**FIG. 2**

100

103a

103

104

160   180   170

103b   104

FIG. 3

FIG. 4

**EP 3 697 245 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1970671 A
- WO 2011157870 A
- US 20170241797 A
- US 20170188950 A
- US 2017303827 A
- US 2016324445 A1